# EUROPÄISCHE PATENTSCHRIFT

(11) **EP 0 680 326 B1**
(45) Veröffentlichungstag und Bekanntmachung des Hinweises auf die Patenterteilung: **27.12.1996**
(21) Anmeldenummer: 94905025.6
(22) Anmeldetag: 10.01.1994
(51) Int. Cl.: A61K 31/55

(54) **PHARMAZEUTISCHE FORMULIERUNG ZUR BEHANDLUNG DER NICOTINABHÄNGIGKEIT**
PHARMACEUTICAL COMPOSITION FOR TREATING NICOTINE DEPENDENCE
FORMULATION PHARMACEUTIQUE POUR LE TRAITEMENT DE LA DEPENDANCE A L'EGARD DE LA NICOTINE

(30) Priorität: 23.01.1993 DE 4301782
(43) Veröffentlichungstag der Anmeldung: 08.11.1995
(73) Patentinhaber: LTS LOHMANN Therapie-Systeme GmbH, 56567 Neuwied (DE); HF ARZNEIMITTELFORSCHUNG GmbH & Co. KG, 59368 Werne (DE)
(72) Erfinder: MOORMANN, Joachim Arnold, D-59368 Werne (DE)
(74) Vertreter: Flaccus, Rolf-Dieter, Dr.
(86) Internationale Anmeldenummer: EP9400055
(87) Internationale Veröffentlichungsnummer: WO9416708

(56) Entgegenhaltungen:
- EP-A- 0 449 247
- EP-A- 0 515 301
- EP-A- 0 515 302
- US-A- 4 663 318
- DIALOG INFORMATION SERVICES, FILE 155: MEDLINE 1966-1994, ACCESSION NUMBER 00713205
- CHEMICAL ABSTRACTS, vol. 95, no. 15, 12. Oktober 1981, Columbus, Ohio, US; abstract no. 127180a, F.I.GRUDEV ET AL. 'MECHANISMS OF ALCOHOL ABSTINENCE ACCORDING TO DATA FROM AN ELECTROENCEPHALOGRAPHIC STUDY'
- CHEMICAL ABSTRACTS, vol. 110, no. 1, 2. Januar 1989, Columbus, Ohio, US; abstract no. 708b, 'EFFECT OF GALANTHAMINE, EPIGALANTHAMINE, GALANTHAMINONE ON THE POSTSYNAPTIC N-CHOLINERGIC RECEPTORS'

## Beschreibung

Die vorliegende Erfindung bezieht sich auf pharmazeutische Formulierungen zur Behandlung der Nicotinabhängigkeit.

Insbesondere ist die vorliegende Erfindung auf pharmazeutische Formulierungen und Vorrichtungen gerichtet, durch die Galanthamin oder eines seiner pharmazeutisch annehmbaren Säureadditionssalze zur Behandlung der Nicotinabhängigkeit auf kontrollierte, z.B. kontinuierliche Weise abgegeben werden.

Die Abhängigkeit von Nicotin erfüllt alle von der WHO definierten Kriterien der Drogenabhängigkeit:
- zwanghafter Gebrauch
- psychoaktive Effekte
- Einfluß auf das Verhalten
- stereotype Konsumgewohnheiten
- Abstinenzerscheinungen bei Entzug oder Toleranzentwicklungen

Rauchen ist also keine "schlechte Angewohnheit" und kann nicht in allen Fällen durch Willensstärke allein unterdrückt werden. Pharmakologen haben im Gehirn Nicotin-Rezeptoren gefunden, die die biologische Erklärung dafür sind, daß so viele Raucher trotz starker Motivation und guter psychologischer Unterstützung immer wieder rückfällig werden.

Diese Erkenntnis führte 1975 zu einem völlig neuen Therapieansatz, der Nicotinzufuhr durch Kaugummi. Zunächst begeistert begrüßt, zeigte das System jedoch recht bald Schwachstellen. So wurden der bittere Geschmack und die geringe soziale Akzeptanz des Kaugummis bemängelt. Auch ist es bei diesen Systemen durchaus zu Mißbrauch durch Überdosierung gekommen.

Alle diese Nachteile führten zur Entwicklung von transdermalen Systemen, die Nicotin enthalten, wie sie z.B. in der deutschen Patentschrift DE 36 29 304 und dem US-Patent 4,597,961 beschrieben sind.

Bei der transdermalen Gabe von Nicotin spielt Geschmack keine Rolle, die Anwendung ist unsichtbar, die Abgabe des Wirkstoffs erfolgt ohne orale Ersatzbefriedigung und Plasmaspitzen werden vermieden.

Als Nebenwirkung werden Hautirritationen an den Applikationsstellen beobachtet, Rötungen, leichte Schwellungen und Juckreiz, die in einigen Fällen zur Aufgabe der Therapie führten.

Nachteilig bei dieser Nicotintherapie ist darüber hinaus, daß bei dieser Art der Behandlung der extremen Giftigkeit des Nicotins keine Rechnung getragen wird.

Es besteht daher eine Bedarf an Medikamenten, die die Symptome der Nicotinabhängigkeit sicher unterdrücken, ohne daß aber die therapeutischen Dosen des Wirkstoffs eine Toxizität besitzen, die der des Nicotins vergleichbar ist.

Bisher wurden zur Behandlung der Nicotinabhängigkeit Stoffe aus folgenden Gruppen eingesetzt:
- Naturstoffe ohne Stickstoff, z.B. γ-Pyrone, Citronensäure, Essigsäure, Campher, Glucose, Vitamine, Terpene u.a.
   - Alkaloide, z.B. Lobelin, Coffein und Apocynaceen-Alkaloide
   - tricyclische Antidepressiva wie z.B. Fluoxetin
   - Cloniddin
   - Pyrrolopyrimidin

Schon aus derr Verschiedenartigkeit der Therapieprinzipien erkennt man, daß einn wirksames Medikament zur Behandlung der Nicotinabhängigkeit noch nicht gefunden wurde, das nicht so toxisch ist, wie Nicotin.

Das Dokument Dokl. Bolg. Akad. Nauk.,41 (9), 1988, S.101-103, befaßt sich mit dem pharmakokinetischen Verhalten u.a. des Cholinesterasehemmers Galanthamin- Hydrobromid an Versuchstier und Mensch, beispielsweise anhand der Löslichkeit von aus Rattenhirnmasse gewonnenen Nicotin-Rezeptoren, sowie mit der Wirkung von Galanthamin an M- und N-cholerischen Rezeptoren.

Die Untersuchungen haben mit der Wirksamkeit von Galanthamin auf die Behandlung von Nicotinabhängigkeit beim Menschen nichts zu tun und geben daher einem Fachmann keine Anregung in dieser Richtung.

Das Dokument Dokl. Akad. Nauk. SSSR, 167 (5), 11. April 1966, S. 1197-2000, beschreibt die Wirkung von Cholinestease-Reaktivatoren auf Vergiftungen von Mäusen und Ratten beispielsweise durch Arecolin oder Nicotin oder OPC aufgrund einer Restauration der Cholinesterase-Aktivität wiie Herzrhytmus-Störungen, Spasmen, Muskelkräfmpe etc. Als Wirkstoffe werden u.a. TEPP, Amine, Methylsulphomethylat von methylphosphinischer Säure, GD-42, Carbophos und Galanthamin experimentell eingesetzt. Ein Vergleich mit der Wirksamkeit von Galanthamin auf die Behandlung von Nikotinabhängigkeit beim Menschen ist auch diesem Dokument nicht zu entnehmen.

Aufgabe der Erfindung ist daher die Bereitstellung eines Arzneistoffes in einer oralen, transdermalen oder anderweitig parenteralen Formulierung, die diesen Arzneistoff möglichst kontrolliert freisetzt und gewährleistet, daß das Verlangen nach Nicotin gemindert wird. Der Begriff parenteral soll also alle Anwendungsformen, außer der oralen, umfassen wie die rektale, intravenöse, interamuskuläre, iintraperitoneale und nasale Anwendung.

Diese Aufgabe wird erfindungsgemäß in überraschender Weise gelöst durch eine Formulierung zur Behandlung der Nicotinabhängigkeit, dadurch gekennzeichnet, daß sie eine wirksame Menge des Wirkstoffs Galanthamin (4a, 5, 9, 11, 12-Hexahydro-3-methoxy-11-methyl-6H-benofuro [3a, 3, 2-ef] [2] benzazepin-6-ol) oder eines seiner pharmazeutisch annehmbaren Säureaditionssalze enthält.

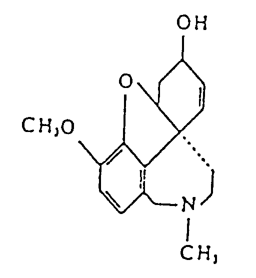

Diese Lösung ist umso erstaunlicher, als Galanthamin zwar ausführlich untersucht und seine pharmakologischen Wirkungen intensiv erforscht worden sind, die erfindungsgemäße Anwendung einer galanthamin-haltigen Formulierung zur Behandlung der Nicotinabhängigkeit aber bisher nicht beschrieben wurde.

Aufgrund seiner pharmakologischen Eigenschaften gehört Galanthamin zur Gruppe der reversibel wirkenden Cholinesterasehemmstoffe, steht in seinen Wirkungen dem Physostigmin und dem Neostigmin nahe, zeichnet sich jedoch durch besondere spezifische Eigenschaften aus. Galanthamin hat eine 3- bis 6-mal größere therapeutische Breite, weil es nicht so toxisch wie Physostigmin oder Neostigmin ist.

Dieser Vorteil wiegt seine auf die Gewichtseinheit bezogene etwas geringere Cholinesterasehemmwirkung auf.

Galanthamin wird in der Medizin für verschiedene Indikationen verwendet, z.B. in der Narkosepraxis zur Aufhebung der Muskelrelaxation nach nichtdepolarisierenden Muskelrelaxantien angewandt. Seine lange Wirkungsdauer macht Galanthamin, das die Eigenschaften von Physostigmin und Neostigmin in sich vereint, zu einem wertvollen Hilfsmittel in der Anaesthesiologie, da viele Patienten nach einer Allgemeinnarkose an einem zentralen anticholinergischen Syndrom leiden. Ferner ist es ein brauchbares Antidot für die Neuroleptanalgesie.

Im Gegensatz zu Neostigmin überwindet Galanthamin die Bluthirnschranke und antagonisiert die cerebralen Wirkungen cholinerger Gifte. Galanthamin erweckt aus dem durch Scopolamin bewirkten Dämmerschlaf.

In der Neurologie wird Galanthmin bei Facialisparesen und anderen Mono- und Polyneuropathien, bei Läbmungsrestzuständen nach Poliomyelitis oder Hirn- bzw. Rückenmarksverletzungen sowie bei der Myasthenia gravis angewandt. In der Augenheilkunde dient Galanthamin zur symptomatischen Behandlung des Engwinkelglaukoms.
Versuchsweise wird Galanthamin bei der Alzheimerschen Krankheit eingesetzt.

Ferner wurde vorgeschlagen, Galanthamin im Rahmen von klinischen Studien zur Alkoholentwöhnung einzusetzen (Opitz, K., DE-PS 40 10 079).

Die Gewinnung des Galanthamins erfolgt z.B. durch Isolierung aus dem kaukasischen Schneeglöckchen Galanthus woronowi Vel., Amaryllidaceae, oder durch Synthese.

Arzneiformen, die Wirkstoffe kontrolliert freisetzen, sind im Stand der Technik bereits bekannt. Die Verabreichung pharmazeutisch wirksamer Verbindungen mittels solcher Formulierungen kann oral, transdermal oder anderweitig parenteral erfolgen. In derartigen Arzneimitteln kann das Galanthamin als solches oder in Form pharmazeutisch annehmbarer Säureadditonssalze vorliegen z.B. als Hydrohalogenid, insbesondere -chlorid oder -bromid, oder als Salz einer anderen pharmazeutisch annehmbaren Säure. Diese Mittel enthalten ferner in der Regel Hilfsstoffe, wie Trägerstoffe, Fließverbesserer, Lösungsmittel und Öle, deren Art und Menge je nach Darbietungsform schwankt. Im allgemeinen liegt der Gehalt an Wirkstoff im Arzneimittel, berechnet als freies Galanthamin, zwischen 0,1 und 50 Gew.-%, vorzugsweise zwischen 2 und 15 Gew.-%.

Einige im Rahmen der vorliegenden Erfindung geeignete Formulierungen zur oralen Verabreichung sollen kurz beschrieben werden.

In einer solchen Formulierung ist der pharmazeutische Wirkstoff z.B. in einer semipermeablen Membran eingekapselt, wie z.B. in Zelluloseacetat. Mit einem Bohrer oder Laser wird in das Kapselmaterial ein winziges Loch gebohrt. Im Körper des Patienten, der behandelt wird, wird durch das Kapselmaterial Wasser absorbiert. Der pharmazeutische Wirkstoff wird durch osmotischen Druck in der gewünschten allmählichen, konstanten und kontrollierten Weise durch die kleine Öffnung getrieben. Solche Systeme sind beispielsweise in den US-Patenten US 3,760,805 und US 3,987,790 beschrieben. In diesen Systemen können die pharmazeutischen Wirkstoffe in fester Form oder absorbiert an Ionenaustauscher-Harze vorliegen.

Ein anderes System zur oralen Verabreichung wird von Sheth und Leeson im US-Patent 4 137 300 beschrieben. Dieses Patent beschreibt eine Formulierung, die eine Wachsmatrix enthält.

Die Wirkstoffe der vorliegenden Erfindung werden mittels entsprechender Formulierungen auf passende und geeignete Weise verabreicht. Die festen Wirkstoffe können in Lösung oder als Suspension verabreicht werden. Das Lösungs- oder Suspensionsmedium kann wäßrig oder organisch sein. Geeignete Lösungs- oder Suspensionsmedien für Galanthamin sind z.B. Wasser, Silikonfluid oder Mineralöl.

Um die Verabreichung einer Verbindung mittels einer Formulierung wie vorstehend beschrieben zu vereinfachen, kann dem System ein Fließverbesserer zugesetzt werden. Einige geeignete Fließverbesserer für orale Formulierungen umfassen beispielsweise Polyethylenglykol, Hydroxypropylmethylcellulose und Zucker.

In einer Formulierung zur transdermalen Verabreichung von Verbindungen gemäß der vorliegenden Erfindung kann der pharmazeutische Wirkstoff in einer Matrix enthalten sein, von der er in der gewünschten allmählichen, konstanten und kontrollierten Weise abgegeben wird. Die Durchlässigkeit der Matrix bei der Freisetzung der Verbindung beruht auf Diffusion. Ein derartiges System ist in dem deutschen Patent 33 15 272 (US 4 769 028) beschrieben. Dieses System besteht aus einer undurchlässigen Deckschicht, einem damit verbundenen, besonders aufgebauten übersättigten Wirkstoff-Reservoir aus einer Polymermatrix, einer mit dem Reservoir verbundenen, für den Wirkstoff durchlässigen Haftklebeschicht und einer die Haftklebeschicht abdeckenden, zum Gebrauch wieder ablösbaren Schutzschicht. Auch Systeme, in denen die Reservoirschicht eine so hohe Eigenklebrigkeit aufweist, daß sie gleichzeitig die Haftklebeschicht darstellt, sind möglich.

Das deutsche Patent DE 38 43 239 (US 5 089 267) beschreibt ein solches System.

Wenn der Wirkstoff durch die Haut absorbiert wird, erhält der zu Behandelnde auf diese Weise einen kontrollierten und vorbestimmbaren Zufluß des Wirkstoffes.

Andere geeignete transdermale Formulierungen sind in den US-Patenten 3,742,951, 3,797,494, 3,996,934 und 4,031,894 beschrieben. Diese Formulierungen bestehen grundsätzlich aus einer Rückschicht, die eine der Oberflächen darstellt, einer für den Wirkstoff durchlässigen Klebschicht, die die andere Oberfläche darstellt und letztlich einem Reservoir, das den Wirkstoff zwischen den beiden die Oberfläche bildenden Schichten enthält. Alternativ dazu kann der Wirkstoff auch in einer Vielzahl von Mikrokapseln enthalten sein, die in der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut oder Schleimhaut des zu Behandelnden steht, kontinuierlich abgegeben. Im Falle von Mikrokapseln kann das Kapselmaterial auch als Membran wirken.

Formulierungen, die zur anderweitigen parenteralen Applikation von Galanthamin und seinen Salzen in Frage kommen, sind solche, die eine Depotwirkung des Wirkstoffs ermöglichen. Hierbei wird die Formulierung als Injektionslösung auf nichtwäßriger Grundlage appliziert. Die möglichen Lösungsmittel sind dem Fachmann bekannt. Als Beispiele seien die vegetabilischen Öle erwähnt, die einzelne Pharmakopöen vorschreiben, wie Erdnußöl, Olivenöl, Mandelöl, Sonnenblumenöl, Sojabohnenöl und Sesamöl. Rizinusöl zeigt oftmals eine besonders günstige Löslichkeit für Arzneimittel; daneben sind auch Öle tierischen Ursprungs geeignet.

Die Öle sind physiologisch indifferent und gut verträglich. Voraussetzung hierfür ist, daß sie besonders gereinigt sind und niedrige Säure- und Peroxidzahlen aufweisen.Da eine intravenöse Applikation wegen der fehlenden Mischbarkeit mit dem Blutserum nicht möglich ist und zu Lungenembolie führen kann, ist ihre Anwendung lediglich für intramuskuläre und subkutane Injektionspräparate möglich. Ölige Lösungen und Suspensionen verbleiben recht lange am Ort der Applikation (oft bis zu 1 Monat) und geben die Wirkstoffe protrahiert frei.

Die Dosierung des Galanthamins oder seiner pharmazeutisch annehmbaren Säureadditionssalze muß so hoch sein und über eine so lange Zeit erfolgen, daß eine nachhaltige Wirkung erzielt wird, und bedarf einer individuellen Einstellung.

Die Erfindung wird durch das folgende Beispiel erläutert:

### Beispiel:

Einfluß von Galanthamin auf das Rauchen von gesunden Probanden.

Im Rahmen der Erprobung eines transdermalen therapeutischen Systems, das in vivo ca. 10 mg Galanthaminbase pro Tag abgibt, wurden neben anderen Probanden auch zwei Raucher als Versuchspersonen herangezogen, da "Rauchen" kein Ausschlußkriterium bei der Prüfung darstellte. Überraschenderweise trat bei den beiden Rauchern das Phänomen auf, daß das Verlangen nach Zigaretten auffällig unterdrückt wurde. Die Tragedauer betrug 24 Stunden. Die Daten sind in der folgenden Tabelle aufgeführt:

**Tab. 1**

| | Zigarettenkonsum ohne Galanthamin | Zigarettenkonsum nach Gabe von 10 mg/Tag Galanthamin transdermal |
|---|---|---|
| Männlicher Proband | 15-20 Zigaretten/Tag | keine |
| Weiblicher Proband | durchschnittl. 60 Zigaretten/Tag | 7 Zigaretten/Tag |

Wie die Tabelle zeigt, bewirkte schon die einmalige Gabe von 10 mg Galanthamin/Tag eine beträchtliche Reduktion des Zigarettenkonsums.

## Patentansprüche

1. Verwendung von Galanthamin oder einem seiner pharmazeutisch annehmbaren Säureadditionssalze zur Herstellung einer pharmazeutischen Formulierung zur Behandlung der Nicotinabhängigkeit.

2. Verwendung nach Anspruch 1, dadurch gekennzeichnet, daß der Wirkstoff in einer oral applizierbaren Form vorliegt.

3. Verwendung nach Anspruch 1, dadurch gekennzeichnet, daß der Wirkstoff in einer parenteral applizierbaren Form vorliegt.

4. Verwendung nach Anspruch 3, dadurch gekennzeichnet, daß der Wirkstoff in einer transdermal applizierbaren Form vorliegt.

5. Verwendung nach einem oder mehreren der Ansprüche 1-4, dadurch gekennzeichnet, daß der Wirkstoff in Anteilen von 0,1-50 Gew.-%, vorzugsweise 2-15 Gew.-%, berechnet als freies Galanthamin im Arzneimittel, enthalten ist.

6. Verwendung nach einem oder mehreren der Ansprüche 1-5, dadurch gekennzeichnet, daß der Wirkstoff mit geeigneten Hilfsstoffen kombiniert ist.

## Claims

1. The use of galanthamine or one of the pharmaceutically acceptable acid addition salts thereof for the production of a pharmaceutical formulation for the treatment of nicotine dependence.

2. The use according to claim 1 characterized in that the active substance is present in an orally applicable form.

3. The use according to claim 1 characterized in that the active substance is present in a parenterally applicable form.

4. The use according to claim 3 characterized in that the active substance is present in a transdermally applicable form.

5. The use according to one or several of claims 1 to 4 characterized in that the active substance is comprised therein in proportions of 0.1-50%-wt., preferably 2-15%-wt., calculated as free galanthamine in the drug.

6. The use according to one or several of claims 1 to 5 characterized in that the active substance is combined with suitable adjuvants.

## Revendications

1. Utilisation de la galanthamine ou d'un de ses sels d'addition d'acides pharmaceutiquement acceptables en vue de la préparation d'une composition thérapeutique destinée au traitement de la nicotinodépendance ou assuétude nicotinique.

2. Utilisation suivant la revendication 1, caractérisée en ce que le principe actif se présente sous une forme applicable par la voie orale.

3. Utilisation suivant la revendication 1, caractérisée en ce que le principe actif se présente sous une forme applicable par la voie parentérale.

4. Utilisation suivant la revendication 3, caractérisée en ce que le principe actif se présente sous une forme applicable par voie transdermique.

5. Utilisation suivant une ou plusieurs des revendications 1 à 4, caractérisée en ce que le principe actif est contenu en proportions de 0,1 à 50% en poids, de préférence, 2 à 15% en poids, calculé sous forme de galanthamine libre, dans le médicament.

6. Utilisation suivant une ou plusieurs des revendications 1 à 5, caractérisée en ce que le principe actif est combiné à des adjuvants appropriés.
